# EUROPEAN PATENT APPLICATION

(11) **EP 1 854 484 A1**
(43) Date of publication of application: **14.11.2007**
(21) Application number: 06713868.5
(22) Date of filing: 16.02.2006
(51) Int. Cl.: A61K 45/00, A61K 31/166, A61K 31/4164, A61K 31/4409, A61K 31/551, A61P 19/00, A61P 19/02

(54) **PAIN REMEDY CONTAINING ROCK INHIBITOR**

(30) Priority: 16.02.2005 JP 2005038539
(71) Applicant: Astellas Pharma Inc., Tokyo 103-8411 (JP)
(72) Inventor: TAKESHITA, Nobuaki, 2-chome, Chuo-ku Tokyo, 1038411 (JP)
(74) Representative: Gille Hrabal Struck Neidlein Prop Roos
(86) International application number: PCT/JP2006/302727
(87) International publication number: WO 2006/088088

(57) **Abstract**

Since a ROCK inhibitor exerts a potent analgesic effect by a single dose after the onset of a cartilage-related disease such as osteoarthritis, and can regenerate or suppress destruction of cartilage tissue and alleviate pain associated with cartilage diseases by multiple doses, administration of an therapeutically effective amount of the ROCK inhibitor to a patient with cartilage-related disease such as osteoarthritis or rheumatoid arthritis can treat the patient with cartilage-related disease and the ROCK inhibitor is thus useful.

## Description

### Technical Field

The present invention relates to treatment of pain using a ROCK inhibitor.

### Background Art

Pain is a subjective combined sensation reflecting an actual or potential tissue damage and an emotional response thereto and exhibits various forms. Pain is classified into somatic pain and psychogenic pain, and the former is further classified into nociceptive pain and neuropathic pain. Nociceptive pain is caused by external stimulation or visceral pathology. Nociceptive pain is mainly acute, which disappears following cure of underlying disease, and plays a role as a biological signal generated by a disorder. Neuropathic pain is chronic pain caused by dysfunction of the peripheral or central nervous system and includes pain due to diabetes, nerve compression and spinal cord injury. Psychogenic pain is chronic pain, which is due to mental disorder rather than physical disorder and cannot be explained by organic disorder, and includes chronic headache, abdominal pain of unknown cause and the like. Chronic pain imparts large distress to patients and is a target of treatment. Especially, chronic pain associated with arthritis, diabetes, cancer and the like requires pain treatment in addition to treatment of underlying disease, but existing analgesics are not satisfactory in terms of efficacy and safety.

Cartilage tissue is composed of chondrocytes and the cartilage matrix and forms the musculoskeletal system together with bones. As diseases related to deformation or destruction of cartilage tissue, many diseases such as osteoarthritis, spondylosis deformans, rheumatoid arthritis are known. Osteoarthritis is a disease in which the joint cartilage is chronically abraded or defected and the form of the joint changes. There are two types of osteoarthritis, primary and secondary osteoarthritis. Primary osteoarthritis is caused by factors such as muscle regression, obesity and mechanical stress, and secondary osteoarthritis is caused by a definite factor such as trauma or some diseases. Rheumatoid arthritis is a disease characterized by chronic arthritis of unknown cause in which inflammation occurs in joint synovial membrane and destruction of cartilages and bones and deformation of joints occur when the disease advances.

Articular cartilage is mainly composed of collagen, proteoglycan, chondrocytes and water. Chondrocytes produce collagen and proteoglycan, which in turn constitute the chondrocyte matrix. Articular cartilage contains no blood vessel, lymphatic vessel or nerve and nutriented by the synovial fluid secreted from the synovial cells. Cartilages are difficult to be repaired, when damaged, and especially hyaline cartilages that cover the joint surface are difficult to be repaired to its original complete form, once damaged. With the advance of these pathologies, surgical treatment such as arthroplasty or joint replacement is required, which imposes excessive burden on patients.

Since cartilage tissue damage is difficult to be repaired and surgical treatment does not provide permanent cure and suffers from concerns about infection, regeneration of cartilage tissue has recently attracted attention as a method of treatment of cartilage-related diseases accompanying deformation and destruction of cartilage tissue. Although studies using high molecular weight proteins such as TCF-β, BMP-2 and concanavalin A have been so far conducted for regeneration of cartilage tissue, no such protein is presently used clinically.

ROCK (Rho kinase) is a serine/threonine kinase that acts downstream of Rho, one of low molecular weight GTP-binding proteins, and is suggested to be involved in various physiological functions such as cell motility, cytoskeleton control, vasoconstriction and inflammation. As the actions of ROCK inhibitor on bone cells and chondrocytes, it has been reported that Y27632 enhances bone formation by bone morphogenetic protein (BMP) (for example, Patent Document 1 and Patent Document 2) and that Y27632 induces in vitro differentiation of chondrocyte progenitor cells to chnrdocytes (for example, Non-patent Document 1). There is no report, however, on efficacy or therapeutic effect of ROCK inhibitors in in vivo models of cartilage lesion/disease (osteoarthritis, rheumatoid arthritis) or in clinical scene.

The analgesic effect of ROCK inhibitors is reported: Y27632 is shown to be involved in hyperalgesia induced by various stimuli (for example, Patent Document 3 and Non-patent Document 2) and H1152 is shown to be involved in pain due to nociceptive stimulus and neurogenic pain (Non-patent Document 3). There is no report, however, showing efficacy of administration after onset of symptom on pain clinically or in an in vivo chronic rheumatism model or osteoarthritis model.
[Patent Document 1] International Publication No. WO00/78351 pamphlet
[Patent Document 2] International Publication No. WO01/17562 pamphlet
[Patent Document 3] International Publication No. WO01/22997 pamphlet
[Non-patent Document1] G. Wang et al, "RhoA/ROCK Signaling Suppresses Hypertrophic Chondrocyte Differentiation", The Journal of Biological Chemistry", 2004, 279, p.13205-13214)
[Non-patent Document 2] (Inoue et al, "Initiation of neuropathic pain requires lysophosphatidic acid receptor signaling", Nature Medicine, 2004, 10, p.712-718)
[Non-patent Document 3] Tatsumi et al, "Involvement of Rho-kinase in Inflammatory and Neuropathic Pain through Phosphorylation of Myristoylated Alanine-Rich C-Kinase Substrate (Marcks)", Neuroscience, 2005, 131, p.491-498)

### Disclosure of the Invention

### Problems to be Solved by the Invention

Presently, as therapeutic agents for cartilage-related diseases, methotrexate, gold compounds, D-penicillamine and the like are used as drugs that suppress the progress of disease, and steroids, NSAIDs, Cox-2 inhibitors and the like are used as anti-inflammatory analgesics. In the treatment of rheumatism, an anti-TNF antibody has been recently shown to have both of an analgesic effect and an effect of suppressing disease progress and attracted attention.

All these agents have only limited therapeutic efficacy, however, and there is a concern about adverse drug reactions following long-term use. Especially, osteoarthritis imposes large burden on patients, since pain continues chronically although it does not accompany potent inflammation. As pain treatment drugs, non-steroidal anti-inflammatory analgesics are mainly used, which are ineffective in many patients and have insufficient efficacy. In addition, these drugs have a problem of adverse drug reactions such as gastrointestinal disorders and cardiovascular risk. Accordingly, drug creation of pain remedies that are highly effective with reduced adverse drug reactions is desired. Further, creation of drugs that suppress destruction of cartilages or promote regeneration of cartilages and suppress progress of disease is strongly demanded.

### Means for Solving the Problems

The present inventors have conducted various tests on ROCK inhibitors to address the problems described above and found that the ROCK inhibitors have a potent analgesic effect on pain in various rat models by single administration after the establishment of pathology (Example 1 and Example 2) and further that the ROCK inhibitors locally regenerate cartilage tissue or suppress cartilage destruction in syndromes in which cartilage damage, cartilage malformation or chondrocytes damage are markedly severe such as osteoarthritis and rheumatoid arthritis (Example 3). Further, as a result of detailed examination of their analgesic effect, the present inventors have obtained a novel finding that various ROCK inhibitors with different structures alleviate pain in osteoarthritis and rheumatoid arthritis without accompanying cartilage protection effect and completed the present invention.

Specifically, the present invention provides the followings.

According to one aspect, the present invention provides a therapeutic agent containing a ROCK inhibitor that treats a patient with pain or a patient with cartilage-related disease.

According to an embodiment, pain associated with the above diseases is alleviated.

According to an embodiment, cartilage tissue is regenerated or destruction of cartilage tissue is suppressed in the cartilage-related diseases.

According to an embodiment, the cartilage-related diseases is osteoarthritis.

According to an embodiment, the ROCK inhibitor is a compound selected from the group consisting of
(+)-(R)-trans-4-(1-aminoethyl)-N-(4-piridyl)cyclohexanecarboxamide,
4[(1R)-1-aminoethyl]-N-(4-piridyl)benzamide,
2-[4-(1H-indazol-5-yl)phenyl]-2-propanamine,
N-(3-methoxybenzyl)-4-(4-piridyl)benzamide,
4-[(trans-4-aminocyclohexyl)amino]-2,5-difluorobenzamide,
4-[(trans-4-aminocyclohexyl)amino]-5-chloro-2-fluorobenzamide and
5-(hexahydro-1H-1,4-diazepin-1-ylsulfonyl)isoquinoline and/or a pharmaceutically acceptable acid addition salt thereof.

Further, in another aspect, the present invention provides a method for treating a patient with pain or a patient with cartilage-related diseases, comprising administering a therapeutically effective amount of a ROCK inhibitor to the patient with the disease to alleviate pain.

According to an embodiment, cartilage tissue is regenerated or destruction of cartilage tissue is suppressed in the cartilage-related diseases.

According to an embodiment, the cartilage-related disease is osteoarthritis.

According to an embodiment, the ROCK inhibitor is a compound selected from the group consisting of
(+)-(R)-trans-4-(1-aminoethyl)-N-(4-piridyl)cyclohexanecarboxamide,
4[(1R)-1-aminoethyl]-N-(4-piridyl)benzamide,
2-[4-(1H-indazol-5-yl)phenyl]-2-propanamine,
N-(3-methoxybenzyl)-4-(4-piridyl)benzamide,
4-[(trans-4-aminocyclohexyl)amino]-2,5-difluorobenzamide,
4-[(trans-4-aminocyclohexyl)amino]-5-chloro-2-fluorobenzamide and
5-(hexahydro-1H-1,4-diazepin-1-ylsulfonyl)isoquinoline and/or a pharmaceutically acceptable acid addition salt thereof.

Further, in another aspect, the present invention provides use of a ROCK inhibitor in manufacturing of a pharmaceutical to treat a patient with pain or a patient with cartilage-related diseases, wherein the pharmaceutical contains an effective amount of the ROCK inhibitor to alleviate pain in the patient with the diseases.

According to an embodiment, cartilage tissue is regenerated or destruction of cartilage tissue is suppressed in the cartilage-related disease.

According to an embodiment, the cartilage-related disease is osteoarthritis.

According to an embodiment, the ROCK inhibitor is a compound selected from the group consisting of
(+)-(R)-trans-4-(1-aminoethyl)-N-(4-piridyl)cyclohexanecarboxamide,
4[(1R)-1-aminoethyl]-N-(4-piridyl)benzamide,
2-[4-(1H-indazol-5-yl)phenyl]-2-propanamine,
N-(3-methoxybenzyl)-4-(4-piridyl)benzamide,
4-[(trans-4-aminocyclohexyl)amino]-2,5-difluorobenzamide,
4-[(trans-4-aminocyclohexyl)amino]-5-chloro-2-fluorobenzamide and
5-(hexahydro-1H-1,4-diazepin-1-ylsulfonyl)isoquinoline and/or a pharmaceutically acceptable acid addition salt thereof.

Analgesics used clinically are expected to exert an immediate effect on pain after onset, since a patient already has had complaint of pain. According to the present invention, a fast-acting analgesic effect of a ROCK inhibitor has been intensively studied and it has been unexpectedly found that a single dose of the ROCK inhibitor is effective for pain in an osteoarthritis model for which a nonsteroidal anti-inflammatory drug (NSAID) is ineffective. The NSAID is ineffective in some patients with osteoarthritis. Although a NSAID (diclofenac) is effective in the early stage of the disease, it is ineffective after establishment of knee joint cartilage damage in the rat MIA-induced osteoarthritis model used for evidencing the present invention. The model can thus be said to well reflect clinical symptoms (J. Vet. Med. Sci., 65, 1195-1199 (2003), Neuroscience lett., 370, 236-240 (2005)). We have unexpectedly found that a single dose of the ROCK inhibitor is also effective even when it is given after establishment of the disease in the model. At that time, a NSAID (diclofenac) was ineffective.

In addition, the present inventors have studied the effect of a ROCK inhibitor on severe cartilage damage and found unexpected effects of the ROCK inhibitor to regenerate cartilage tissue or suppress cartilage destruction in a damaged region and to alleviate pain.

### The Effect of the Invention

The present invention is useful for the treatment of cartilage-related diseases based on the effects of alleviating pain in a cartilage damaged region and further regenerating cartilage tissue or suppressing cartilage destruction. A ROCK inhibitor is highly useful as a therapeutic agent for osteoarthritis, since it exerted efficacy by a single dose after establishment of pathology and alleviated cartilage damage by multiple doses in a MIA-induced osteoarthritis model in which NSAIDs is not effective..

### Best Mode for Carrying Out the Invention

The present invention will be described in detail below.

"ROCK" used herein refers to a serine/threonine kinase that acts downstream of Rho, and ROCK I (also referred to as ROK β or p160ROCK) and ROCK II (also referred to as ROK α or Rho kinase) both affected on RhoA have been so far identified (Mol. Cell. Biol., 4, 446-456 (2003)). "ROCK inhibitor" used in the present invention is a compound that inhibits both or either one of the ROCKS, is not particularly limited, and includes those described in, for example, International Publication No. WO98/06433 pamphlet, International Publication No. WO00/78351 pamphlet, International Publication No. WO01/17562 pamphlet, International Publication No. WO02/076976 pamphlet, International Publication No. WO02/076977 pamphlet, International Publication No. WO03/082808 pamphlet, International Publication No. WO05/035506 pamphlet, International Publication No. WO05/074643 pamphlet and others. Preferably, the ROCK inhibitor used in the present invention is Y27632; (+)-(R)-trans-4-(1-aminoethyl)-N-(4-piridyl)cyclohexanecarboxamide dihydrochloride, or Wf536; 4-[(1R)-1-aminoethyl]-N-(4-piridyl)benzamide monohydrochloride or Fasudil; 5-(hexahydro-1H-1,4-diazepin-1-ylsulfonyl)isoquinoline hydrochloride or Compound 1; 4-[(trans-4-aminocyclohexyl)amino]-2,5-difluorobenzamide or Compound 2; 4-[(trans-4-aminocyclohexyl)amino]-5-chloro-2-fluorobenzamide or Compound 3; 2-[4-(1H-indazol-5-yl)phenyl]-2-propanamine dihydrochloride or Compound 4; N-(3-methoxybenzyl)-4-(4-piridyl)benzamide.

The above compounds may be made as acid addition salts with pharmaceutically acceptable inorganic acids or organic acids, as required. Examples of the acid addition salts include salts with mineral acids such as hydrochloric acid, hydrobromic acid, sulfuric acid, phosphoric acid and the like; salts with organic carboxylic acids such as formic acid, acetic acid, fumaric acid, maleic acid, oxalic acid, citric acid, malic acid, tartaric acid, aspartic acid and glutamic acid; and salts with sulfonic acids such as methanesulfonic acid, benzenesulfonic acid, p-toluenesulfonic acid, hydroxybenzenesulfonic acid, dihydroxybenzene sulfonic acid and the like.

The compounds and acid addition salts thereof may be an anhydride, hydrate or solvate thereof.

"Cartilage-related disease" that can be treated using the present invention include any cartilage-related disease (for example, osteoarthritis and rheumatoid arthritis) having cartilage damage, abnormal cartilage formation and/or chondrocyte disorder, and especially, osteoarthritis can be effectively treated by the method of the present invention.

Administration routes of the ROCK inhibitor used in the method of the present invention include, but are not limited to, oral administration, intra-articular administration, subcutaneous administration, intravenous administration, intranasal administration, percutaneous administration and combinations thereof.

When the ROCK inhibitor of the present invention is used as a pharmaceutical composition, it is also desirable to use the ROCK inhibitor as a part of a formulated preparation. These pharmaceutical compositions contain the ROCK inhibitor in a mixture with at least one or several suitable organic or inorganic carriers or excipients or other pharmacological therapeutic agents, and used in forms of, for example, solid, semi-solid and liquid pharmaceutical preparations. The active ingredient may be mixed with, for example, pharmaceutically commonly-used non-toxic carriers to form into granules, tablets, pellets, lozenges, capsules, suppositories, creams, ointments, aerosols, inhalation powders, and liquid forms such as injection liquids, emulsions and suspensions; orally ingestable forms; eye drops; and any other form suitable for its use. If required, auxiliary substances such as stabilizing agents, thickeners, wetting agents, hardening agents and colorants; fragrance and buffers; and any other commonly used additives may be added to the pharmaceutical preparation.

The effective amount of the ROCK inhibitor of the present invention for the treatment of cartilage-related diseases differs according to the age and condition of a patient and depends on the type of formulation and mode of administration of the therapeutic agent, the stage of disease and administration interval. The ROCK inhibitor is administered, however, generally at a dose ranging from about 0.0001 to 1000 mg, preferably at a dose ranging from 0.001 to 500 mg, further preferably at a dose ranging from 0.01 to 100 mg per day. The dose may be smaller or larger than the above range as required, however, considering various conditions.

An in vivo rat disease model assay will be described as Examples to illustrate the present invention in detail, but the present invention is not limited by these

### Examples.

### [Examples]

### (Example 1)

### (Therapeutic Effect of the ROCK inhibitor in Monoiodoacetate (MIA)-induced Osteoarthritis Model: Analgesic Test by Assessment of Hindlimb Weight Distribution)

This disease model was prepared according to the description in Toxicol Pathol 31(6), 619-624 (2003). Female SD rats (6-7 weeks old, Charles River Laboratories Japan, Inc.) were anesthetized with halothane (Takeda Pharmaceutical Company, Limited) and given a single intra-articular injection of 1 mg of monosodium iodoacetate (MIA; Sigma, St. Louis) through the infrapatellar ligament of the right knee. MIA was dissolved with physiological saline and administered in a volume of 50 uL using a 27-gauge, 0.5-inch needle. Three weeks after the injection of MIA (establishment of pathology of osteoarthritis), single oral administration of a ROCK inhibitor or diclofenac was given and the weights of both hindlimbs of the rats were determined using an incapacitance tester (Linton Instrumentation, Norfork, UK) 2 hours after administration of the compounds to obtain ED₅₀ values (Table 1). Following administration of MIA, the difference in weight between the right and left hindlimbs was about 40 g on average.

**[Table 1]**

| ROCK inhibitor | Difference in weight between the right and left hindlimbs of rats: ED₅₀ (mg/kg) |
|---|---|
| Wf536 | <30 |
| Fasudil | <10 |
| Compound 1 | <10 |
| Compound 2 | <10 |
| Compound 3 | <10 |
| Compound 4 | <10 |
| Diclofenac (NSAIDS) | >30 |

### (Example 2)

### (Pain Alleviation Effect of the Rock Inhibitor on Bradykinin-induced Joint Pain Model)

Preparation of the joint pain model and evaluation of a level of pain were conducted based on the description in Folia pharmacol. japon. 92, 17-27 (1988). Female SD rats (6-7 weeks old, Charles River Laboratories Japan, Inc.) were anesthetized with halothane (Takeda Pharmaceutical Company, Limited) and given a single intra-articular injection of a saline solution of Wf536 or Compound 2 into the knee joint cavity of the hindlimb using a 27-gauge, 0.5-inch needle at a dose of 0.03 µg/site, 0.3 µg/site or 3 µg/site. Thirty minutes after administration of the drug, a physiological saline solution of bradykinin was injected into the knee joint cavity of the hindlimb (3 µM/site/50 µl), then the response to pain of the rat after administration of bradykinin was observed. The level of pain was scored as five grade point (0-4) as follows: 0: no lameness to lameness for 10 seconds; 1: lameness for 10 to 30 seconds; 2: lift of the limb within 10 seconds or lameness for 31 or more seconds; 3: three-legged gait within 10 seconds followed by lameness; and 4: three-legged gait for 10 or more seconds followed by lameness. About 10 rat models were used in each experimental group. It was confirmed that administration of bradykinin leads to response to pain scored as about 3. Administration of Wf536 dose-dependently improved the response to pain, and especially the pain score significantly decreased from 2.6 to 1.2 following administration at 3 µg/site. When Compound 2 was administered at 0.3 µg/site, the pain score significantly decreased from 2.6 to 1.1 to show significant improvement.

### (Example 3)

### (Therapeutic Effect of the ROCK Inhibitor in Monoiodoacetate (MIA)-induced Osteoarthritis Model)

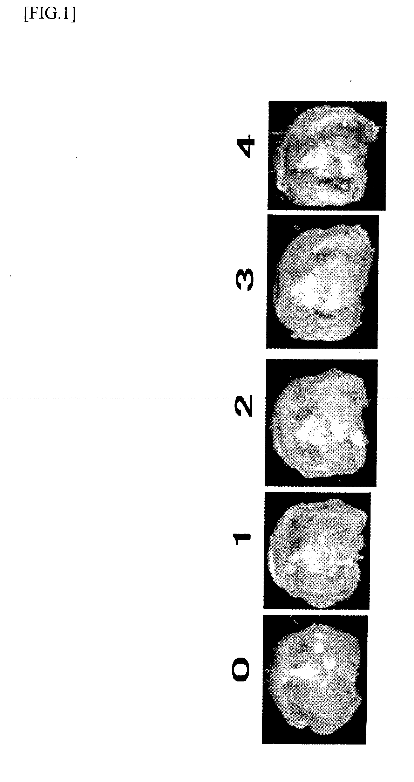

This disease model was prepared according to the description in Toxicol Pathol 31 (6), 619-624 (2003). Female SD rats (6-7 weeks old, Charles River Laboratories Japan, Inc.) were anesthetized with halothane (Takeda Pharmaceutical Company, Limited) and given a single intra-articular injection of 1 mg of monosodium iodoacetate (MIA; Sigma, St. Louis) through the intrapatellar ligament of the right knee. MIA was dissolved with physiological saline and administered in a volume of 50 µL using a 27-gauge, 0.5-inch needle. Three weeks after the injection, a tibia was isolated and morphological change of the distal end of the tibia was scored (0-4; Figure 1). About 8 rats were used for each group. Three weeks after the injection of MIA, cartilage damage of about score 3 was confirmed. Wf536 or Compound 2 was injected intra-articularly at a concentration of 0.03 µg/site, 0.3 µg/site or 3 µg/site 2 times a week (every 2 to 3 days) from the day of administration of MIA. Three weeks after the injection, administration of Wf536 dose-dependently improved the MIA-induced cartilage damage, and especially with administration at 3 µg/site the score significantly decreased from 3 to 2.2 and thus improved. With administration of Compound 2 at 0.3 µg/site, the score significantly decreased from 3 to 2.3 and thus improved.

### Brief Description of the Drawings

[FIG. 1]
   Figure 1 shows scores (0 to 4) of morphological changes of the distal end of the tibia in the MIA-induced osteoarthritis model. 0: no damage; 1: damage on the surface of cartilage; 2: cartilage degradation; 3: cartilage degradation and subchondral bone damage; 4: cartilage degradation, subchondral bone damage and spur formation.

## Claims

1. A therapeutic agent for a patient with pain, comprising a ROCK inhibitor with an effective amount to alleviate pain in the patient with the disease.

2. The therapeutic agent according to claim 1, wherein the pain is pain associated with cartilage-related disease.

3. A therapeutic agent for cartilage-related disease, comprising a ROCK inhibitor with an effective amount to regenerate cartilage tissue or suppress destruction of cartilage tissue of a patient with the disease.

4. The therapeutic agent according to claim 2 or 3, wherein the cartilage-related disease is osteoarthritis.

5. The therapeutic agent according to claim 4, wherein the ROCK inhibitor is a compound selected from the group consisting of
(+)-(R)-trans-4-(1-aminoethyl)-N-(4-piridyl)cyclohexanecarboxamide,
4[(1R)-1-aminoethyl]-N-(4-piridyl)benzamide,
2-[4-(1H-indazol-5-yl)phenyl]-2-propanamine,
N-(3-methoxybenzyl)-4-(4-piridyl)benzamide,
4-[(trans-4-aminocyclohexyl)amino]-2,5-difluorobenzamide,
4-[(trans-4-aminocyclohexyl)amino]-5-chloro-2-fluorobenzamide and 5-(hexahydro-1H-1,4-diazepin-1-ylsulfonyl)isoquinoline and/or a pharmaceutically acceptable acid addition salt thereof.

6. The therapeutic agent according to claim 5, wherein the ROCK inhibitor is a compound selected from the group consisting of
4[(1R)-1-aminoethyl]-N-(4-piridyl)benzamide and
4-[(trans-4-aminocyclohexyl)amino]-5-chloro-2-fluorobenzamide and/or a pharmaceutically acceptable acid addition salt thereof.

7. A method of treating a patient with pain, comprising administering an effective amount of a ROCK inhibitor to the patient with the disease to alleviate pain.

8. The method according to claim 7, wherein the pain is pain based on cartilage-related disease.

9. A method of treating a patient with cartilage-related disease, comprising administering an effective amount of a ROCK inhibitor to the patient with the disease to regenerate cartilage tissue or suppress destruction of cartilage tissue.

10. The method according to claim 8 or 9, wherein the cartilage-related disease is osteoarthritis.

11. The method according to claim 10, wherein the ROCK inhibitor is a compound selected from the group consisting of
(+)-(R)-trans-4-(1-aminoethyl)-N-(4-piridyl)cyclohexanecarboxamide,
4[(1R)-1-aminoethyl]-N-(4-piridyl)benzamide,
2-[4-(1H-indazol-5-yl)phenyl]-2-propanamine,
N-(3-methoxybenzyl)-4-(4-piridyl)benzamide,
4-[(trans-4-aminocyclohexyl)amino]-2,5-difluorobenzamide,
4-[(trans-4-aminocyclohexyl)amino]-5-chloro-2-fluorobenzamide and 5-(hexahydro-1H-1,4-diazepin-1-ylsulfonyl)isoquinoline and/or a pharmaceutically acceptable acid addition salt thereof.

12. The method according to claim 11, wherein the ROCK inhibitor is a compound selected from the group consisting of 4[(1R)-1-aminoethyl]-N-(4-piridyl)benzamide and 4-[(trans-4-aminocyclohexyl)amino]-5-chloro-2-fluorobenzamide and/or a pharmaceutically acceptable acid addition salt thereof.

13. Use of a ROCK inhibitor in manufacturing a pharmaceutical for treating pain, wherein the pharmaceutical comprises an effective amount of the ROCK inhibitor to alleviate pain in a patient with the disease.

14. The use according to claim 13, wherein the pain is pain based on a cartilage-related disease.

15. Use of a ROCK inhibitor in manufacturing of a pharmaceutical for treating cartilage-related disease, wherein the pharmaceutical comprises an effective amount of the ROCK inhibitor to regenerate cartilage tissue or suppress destruction of cartilage tissue in a patient with the disease.

16. The use according to claim 14 or 15, wherein the cartilage-related disease is osteoarthritis.

17. The use according to claim 16, wherein the ROCK inhibitor is a compound selected from the group consisting of
(+)-(R)-trans-4-(1-aminoethyl)-N-(4-piridyl)cydohexanecarboxamide,
4[(1R)-1-aminoethyl]-N-(4-piridyl)benzamide,
2-[4-(1H-indazol-5-yl)phenyl]-2-propanamine,
N-(3-methoxybenzyl)-4-(4-piridyl)benzamide,
4-[(trans-4-aminocyclohexyl)amino]-2,5-difluorobenzamide,
4-[(trans-4-aminocyclohexyl)amino]-S-chloro-2-fluorobenzamide and 5-(hexahydro-1H-1,4-diazepin-1-ylsulfonyl)isoquinoline and/or a pharmaceutically acceptable acid addition salt thereof.

18. The use according to claim 17, wherein the ROCK inhibitor is a compound selected from the group consisting of 4[(1R)-1-aminoethyl]-N-(4-piridyl)benzamide and 4-[(trans-4-aminocyclohexyl)amino]-5-chloro-2-fluorobenzamide and/or a pharmaceutically acceptable acid addition salt thereof.
